# EUROPÄISCHE PATENTSCHRIFT

(11) **EP 0 915 337 B1**
(45) Veröffentlichungstag und Bekanntmachung des Hinweises auf die Patenterteilung: **19.07.2006**
(21) Anmeldenummer: 98117637.3
(22) Anmeldetag: 17.09.1998
(51) Int. Cl.: G01N 31/00, G01N 33/00

(54) **Verfahren und Vorrichtung zum kontinuierlichen Nachweis von heteroatomhaltigen organischen Verbindungen, die in einer gasförmigen Phase vorliegen**
Process and device for continuous detection of heteroatom containing organic conpounds in a gaseous phase
Procédé et dispositif pour la détection continue des composés organiques contenant des hétéroatomes en phase gazeuse

(30) Priorität: 06.11.1997 DE 19749012
(43) Veröffentlichungstag der Anmeldung: 12.05.1999
(73) Patentinhaber: Degussa AG, 40474 Düsseldorf (DE)
(72) Erfinder: Duve, Hans, 48249 Dülmen (DE)

(56) Entgegenhaltungen:
- WO-A-97/38304
- DE-A- 3 909 227
- GB-A- 2 174 364

## Beschreibung

Die Erfindung betrifft ein Verfahren zum kontinuierlichen Nachweis von heteroatomhaltigen organischen Verbindungen, auch wenn diese nur in äußerst geringen Konzentrationen in einer gasförmigen Phase vorliegen, sowie eine geeignete Vorrichtung zur Durchführung des Verfahrens.

Der Nachweis von heteroatomhaltigen organischen Verbindungen in gasförmigen Phasen und/oder Gasströmen auch in äußerst geringen Konzentrationen kann z. B. aus technischen Gründen oder zum Schutz der Umwelt von hohem Interesse sein, wenn die zu überwachenden Verbindungen störende Auswirkungen auf z. B. Prozeßabläufe oder die Umwelt haben können. Heteroatomhaltige organische Verbindungen sind organische Verbindungen, die organisch gebundene Heteroatome wie z. B. Chlor oder Fluor aufweisen.

In DE-39 09 227 wird ein Verfahren zum Nachweis von zersetzungsfähigen organischen Verbindungen, die in einer gasförmigen Phase vorliegen, beschrieben, das in einer bevorzugten Ausführung auch zum kontinuierlichen Nachweis von heteroatomhaltigen organischen Verbindungen in den zu untersuchenden Gasströmen eingesetzt werden kann. Bei dieser Verfahrensausführung wird die zu untersuchende gasförmige Phase in eine begrenzte Wassermenge eingebracht, die im Kreis durch einen UV-Reaktor, einen Ionendetektor und anschließend über einen Ionenaustauscher gefahren wird. Durch die UV-Bestrahlung des Kreislaufwassers werden die eingebrachten organischen Verbindungen in Kohlendioxid (CO₂) und - zumindest beim Vorhandensein von heteroatomhaltigen organischen Verbindungen - in nicht kohlenstoffhaltige Zersetzungsprodukte wie z. B. Chlorid- oder Fluorid-Ionen aus organisch gebundenen Chlor- oder Fluor-Heteroatomen zersetzt. Beim Verfahren nach DE-39 09 227 werden auch diese nicht kohlenstoffhaltigen Zersetzungsprodukte durch den Ionendetektor im Kreislaufwasser nachgewiesen.

Es ist ferner bekannt, daß sich die Nachweisempfindlichkeit des Verfahrens nach DE-39 09 227 erhöhen läßt, wenn pro Zeiteinheit zwar eine konstante Menge zersetzungsfähiger heteroatomhaltiger organischer Verbindungen über die gasförmige Phase in die begrenzte Wassermenge eingebracht wird, gleichzeitig aber die Umlaufgeschwindigkeit des Kreislaufwassers gedrosselt wird, so daß es zu einer Anreicherung von nicht kohlenstoffhaltigen Zersetzungsprodukten im Wasser des UV-Reaktors und nachfolgend am Ionendetektor kommt. Gemäß DE-39 09 227 werden so Nachweisgrenzen im ppt-Bereich (ppt = parts per trillion, 1 : 10¹²) erzielt.

Es wurde jedoch beobachtet, daß das fortgesetzte Absenken der Umlaufgeschwindigkeit des Kreislaufwassers zu einem Betriebspunkt führt, ab dem ein überproportionaler Anstieg des Meßwertes am Ionendetektor im Kreislaufwasser zu beobachten ist, der nicht mehr allein auf den Gehalt an heteroatomhaltigen organischen Verbindungen in der Gasphase und auf die Anreicherung ihrer nicht kohlenstoffhaltigen Zersetzungsprodukte im Kreistaufwasser zurückzuführen ist. Das Verfahren nach DE-39 09 227 hat an diesem Punkt seine Nachweisgrenze und die Grenze seiner Einsatztauglichkeit erreicht, da nicht mehr sicher aus dem Meßwert des Ionendetektors auf heteroatomhaltige organische Verbindungen in der gasförmigen Phase rückgeschlossen werden kann.

Damit stellt sich die Aufgabe, das Verfahren nach DE-39 09 227 zu modifizieren und - neben einer hierzu geeigneten Vorrichtung - ein einfaches Verfahren zu finden, mit dem man die Nachweisgrenze für heteroatomhaltige organische Verbindungen in gasförmigen Phasen weiter herabsetzen kann, ohne zugleich die Betriebssicherheit des Nachweises zu gefährden.

Diese Aufgabe wird erfindungsgemäß gelöst gemäß Patentanspruch 1 durch ein Verfahren zum kontinuierlichen Nachweis von zersetzungsfähigen heteroatomhaltigen organischen Verbindungen, die in einer gasförmigen Phase vorliegen, bei dem das zu untersuchende Probegut in eine begrenzte Wassermenge eingebracht wird, die Bestandteil eines Wasserkreislaufs ist, der Kreislaufwasser über einen UV-Reaktor, in dem die zersetzungsfahigen heteroatomhaltigen organischen Verbindungen aus dem Probegut bestrahlt werden, über einen Ionendetektor, der zumindest die ionogenen Zersetzungsprodukte nachweist, und über einen Ionenaustauscher führt, das dadurch gekennzeichnet ist, daß aus dem Wasserkreislauf ein Teilstrom des Kreislaufwassers vor dem Eintritt in den UV-Reaktor abgezweigt, am UV-Reaktor und Ionendetektor vorbeigeführt und vor dem oder am Eintritt in den Ionenaustauscher wieder mit dem restlichen Kreislaufwasser vereinigt wird.

Gegenstand der Erfindung ist darüber hinaus gemäß Patentanspruch 10 eine geeignete Vorrichtung zur Durchführung des erfindungsgemäßen Verfahrens.

Überraschenderweise wurde nämlich gefunden, daß Ionenaustauscher einer Mindestdurchlaufgeschwindigkeit der zu entionisierenden Flüssigkeit bedürfen, da sie ansonsten bei Unterschreiten dieses Mindestwertes wieder Ionen bzw. organische Stoffe an die Flüssigkeit abgeben können, anstatt sie von Ionen zu befreien. Die erforderliche Mindestdurchflußgeschwindigkeit richtet sich dabei nach dem Beladungszustand des Ionenaustauschers und nach den daraus resultierenden (dynamischen) Stoffgleichgewichten zwischen Ionenaustauschermaterial und flüssiger Phase beim Durchlaufen des Ionenaustauschers. Die Mindestdurchflußgeschwindigkeit kann bei Bedarf empirisch in Abhängigkeit vom Beladungszustand ermittelt werden.

Mit Hilfe des erfindungsgemäßen Verfahrens wird sichergestellt, daß die Mindestdurchflußgeschwindigkeit des Kreislaufwassers durch den Ionenaustauscher, der das für die Analyse als Transportmittel, Lösemittel und Reaktionspartner benötigte Wasser mit extrem hoher Reinheit stets wieder für den Kreislaufbetrieb verfügbar macht, auch bei Drosselung der Durchflußmenge durch den UV-Reaktor nicht unterschritten wird. Erfindungsgemäß ist hierzu zumindest eine Bypass-Leitung vorgesehen, die einen Teil des Kreislaufwassers, das den Ionenaustauscher durchströmt, am UV-Reaktor und Ionendetektor vorbeiführt. Dadurch wird eine Drosselung der Durchflußmenge durch den UV-Reaktor und durch den Ionendetektor und eine Steigerung des Anreicherungseffektes möglich, ohne den Ionenaustauscher bezüglich der Durchflußmenge und damit in seiner Wirkung zu beeinträchtigen. Im folgenden wird die Erfindung näher beschrieben.

Der UV-Reaktor ist erfindungsgemäß - wie aus DE-39 09 227 bekannt - in einen Wasserkreislauf integriert und mit einer begrenzten Menge des Kreislaufwassers gefüllt. Der externe Wasserkreislauf wird mit einer Pumpe umgewälzt. Kreislaufwasser durchläuft also ständig den UV-Reaktor, wobei die im Kreislaufwasser enthaltenen organischen Verbindungen photochemisch zersetzt und ionisiert werden. Der Gehalt des Kreislaufwassers an Ionen kann im Kreislaufwasser, das den UV-Reaktor verläßt, mit einem Ionendetektor nachgewiesen werden. Weil das Kreislaufwasser vor dem Rückführen eines Teils des Wassers in den UV-Reaktor über einen Ionenaustauscher geführt wird, werden die im Kreislaufwasser enthaltenen Ionen kontinuierlich aus diesem entfernt. Durch einen niedrigen und konstanten Meßwert am Ionendetektor hinter dem UV-Reaktor wird z. B. angezeigt, daß das den UV-Reaktor verlassende Kreislaufwasser frei ist von Zersetzungsprodukten heteroatomhaltiger organischer Verbindungen.

Die zu untersuchende gasförmige Phase kann in einer bevorzugten Ausgestaltung des erfindungsgemäßen Verfahrens in den unteren Teil des UV-Reaktors in das darin befindliche Kreislaufwasser, welches den UV-Reaktor ständig durchfließt, eingeperlt werden. Im oberen Teil des UV-Reaktors kann die gasförmige Phase ggf. den Reaktor verlassen, wogegen der Teilstrom des Kreislaufwassers, der durch den UV-Reaktor fließt, über den Ionendetektor geführt wird, mit dem am UV-Reaktor und am Ionendetektor vorbeigeführten Kreislaufwasser vereint wird und über einen Ionenaustauscher mit Hilfe einer im Kreislauf vorhandenen Pumpe geführt wird, bevor er als Teilstrom erneut in den unteren Teil des UV-Reaktors rückgeführt wird. Im Wasserkreislauf kann darüber hinaus ggf. ein Kreislaufwasservorratsgefäß vorhanden sein. Die über die gasförmige Phase in den UV-Reaktor eingebrachten heteroatomhaltigen organischen Verbindungen werden unter Einfluß der UV-Bestrahlung in Kohlendioxid und nicht kohlenstoffhaltige Zersetzungsprodukte umgewandelt. Die nicht kohlenstoffhaltigen Zersetzungsprodukte (z. B. Chlorid-, Fluorid-Ionen) verbleiben vollständig im Kreislaufwasser und können mit dem Ionendetektor ggf. ionensensitiv nachgewiesen werden. Der Gehalt des Kreislaufwassers an Kohlendioxid im Ablauf des UV-Reaktors ist von verschiedenen Faktoren abhängig. In der den UV-Reaktor ggf. verlassenden Gasphase kann das bei der Zersetzung der organischen Verbindungen entstandene Kohlendioxid mit einem Kohlendioxid-Detektor nachgewiesen werden.

Wird pro Zeiteinheit eine konstante Menge zersetzungsfähiger heteroatomhaltiger organischer Verbindungen über die gasförmige Phase in das im UV-Reaktor befindliche Wasser eingebracht, kann nach DE-39 09 227 durch eine Verringerung der durch den UV-Reaktor geleiteten Kreislaufwassermenge pro Zeiteinheit der Gehalt an nicht kohlenstoffhaltigen Zersetzungsprodukten im Wasser des UV-Reaktors angereichert werden. Wie oben beschrieben, ist dieser Anreicherungseffekt beim Verfahren nach DE-39 09 227 dadurch beschränkt in seiner Anwendbarkeit, daß ab einem gewissen Punkt die Durchflußgeschwindigkeit des Kreislaufwassers durch den Ionenaustauscher so niedrig ist, daß der Ionenaustauscher wieder Ionen bzw. organische Stoffe an das Wasser abgibt. Zur Aufrechterhaltung einer ausreichenden Mindestdurchflußgeschwindigkeit des Kreislaufwassers durch den Ionenaustauscher wird daher erfindungsgemäß das Kreislaufwasser in zumindest zwei Teilströme vor dem UV-Reaktor aufgeteilt, die später noch vor dem Durchlaufen des Ionenaustauschers wieder vereinigt werden. Zur Aufteilung des Kreislaufwassers auf die Teilströme befindet sich ein Regulierventil vorzugsweise im Teilstrom über den UV-Reaktor. Bevorzugt sind in allen Teilstromleitungen Ventile zur Volumenstromeinstellung angeordnet.

Ausgehend von der bevorzugten Ausführung mit zwei Teilströmen wird der erste Teilstrom über den UV-Reaktor und den Ionendetektor geleitet, während der zweite Teilstrom in bezug auf UV-Reaktor und Ionendetektor einen Bypass darstellt. Der Volumenstrom des ersten Teilstroms kann so, je nach erforderlichem Anreicherungseffekt zum Erzielen einer hohen Nachweisempfindlichkeit des erfindungsgemäßen Verfahrens, beliebig reduziert werden, wobei der zweite Teilstrom bei entsprechend steigendem Volumenstrom als Bypass für das übrige Kreislaufwasser dient. Da beide Teilströme spätestens vor dem Ionenaustauscher wieder vereint werden, kann so trotz der Reduzierung der Durchflußgeschwindigkeit durch den UV-Reaktor die Durchflußgeschwindigkeit durch den Ionenaustaucher konstant oder annähernd konstant bzw. ausreichend hoch gehalten werden. Auf diese Weise läßt sich der Anreicherungseffekt nicht kohlenstoffhaltiger, ionogener Zersetzungsprodukte in dem den UV-Reaktor verlassenden Kreislaufwasser über das aus DE-39 09 227 bekannte Maß hinaus steigern, so daß erfindungsgemäß Nachweisgrenzen im niedrigen ppt-Bereich bezogen auf den Gehalt an heteroatomhaltigen organischen Verbindungen in der gasförmigen Phase erzielt werden können.

Bevorzugt sollte die in den UV-Reaktor eingeleitete gasförmige Phase frei von Kohlendioxid sein oder vor dem Einleiten mit einem Kohlendioxid-Adsorber von Kohlendioxid befreit werden. In diesem Falle wird das bei der Zersetzung der organischen Verbindungen entstandene Kohlendioxid fast vollständig aus dem Kreislaufwasser entfernt . Als Kohlendioxid-Adsorber sind Natronkalk und Natronasbest geeignet. Werden als Kohlendioxid-Adsorber gekörnter Natronkalk oder Natronkalk-Plätzchen eingesetzt, ist es zweckmäßig, diese vorher mit demineralisiertem Wasser anzufeuchten bzw. auszuwaschen. Durch die Behandlung mit Wasser wird die Adsorption von organischen Kohlenstoffverbindungen aus dem Trägergas verhindert. Diese Eigenschaft der Natronkalk-Plätzchen bleibt überraschenderweise nach dem Abtrocknen im Trägergasstrom erhalten.

Wird die zu untersuchende Gasphase gemäß einer anderen bevorzugten Verfahrensausführung durch das Wasser eines ggf. im Kreislauf befindlichen Vorratsgefäßes geleitet, lösen sich alle in der Gasphase vorhandenen Verbindungen proportional zu ihrem Verteilungskoeffizienten (flüssige Phase/gasförmige Phase) in diesem Wasser. Für jeden Inhaltsstoff der Gasphase stellt sich im Wasser des Vorratsgefäßes ein dynamisches Gleichgewicht ein, also auch für ionenbildende Inhaltsstoffe. Wird das Kreislaufwasser, das das Vorratsgefäß verläßt, anschließend über den Ionenaustauscher geleitet, werden alle aus der Gasphase gelösten ionenbildenden Bestandteile (z. B. CO₂, NH₃, HCl) zusammen mit den ionogenen Zersetzungsprodukten aus dem Kreislaufwasser entfernt, bevor dieses zumindest teilweise durch den UV-Reaktor geleitet wird. Alle bei der Zersetzung der heteroatomhaltigen organischen Verbindungen im UV-Reaktor entstandenen Produkte verbleiben zunächst in diesem Teilstrom und können mit dem Ionendetektor im Kreislaufwasser hinter dem UV-Reaktor nachgewiesen werden.

Enthält die zu untersuchende Gasphase Verbindungen, die in wäßriger Lösung ohne UV-Bestrahlung Ionen bilden, und werden diese Verbindungen beim Durchleiten der Gasphase durch das Wasser im Vorratsgefäß vollständig aus der Gasphase entfernt, dann kann es vorteilhaft sein, die das Vorratsgefäß verlassende Gasphase auch durch das Wasser des UV-Reaktors zu leiten. Durch ein Anordnen des Ionenaustauschers nach dem Vorratsgefäß können auch hier ionogene Bestandteile vor dem UV-Reaktor aus dem Kreislaufwasser entfernt werden. In diesem Falle können wiederum die entstandenen Zersetzungsprodukte in der den UV-Reaktor verlassenden Gasphase und im Kreislaufwasser hinter dem UV-Reaktor nachgewiesen werden.

Die Umwälzpumpe zur Zirkulation des Kreislaufwassers ist vorzugsweise dem Ionenaustauscher nachgeschaltet; es bietet sich jedoch an, diese in jedem Fall nach der Vereinigung der Teilströme und vor der Aufteilung des Kreislaufwasser in Teilströme anzuordnen. Der Volumenstrom dieses Hauptstroms kann über die Pumpe eingestellt werden. Zur Aufteilung des Kreislaufwassers auf die Teilströme sind in die Teilstromleitungen vorzugsweise Ventile integriert, die eine exakte Einstellung der Größe der Teilströme ermöglichen. Bevorzugt befindet sich in der Teilstromleitung, die über den UV-Reaktor führt, ein Volumenstrommesser zur Volumenstromkontrolle. Der Mindestwert der Durchflußmenge über den Ionenaustauscher wird vorzugsweise so bemessen, daß der Ionenaustauscher im Hauptstrom bis zu seiner Grenzbeladung stets funktionsfähig bleibt und keine Ionen bzw. organischen Stoffe an das Kreislaufwasser abgibt.

Durch das Absperren des Teilzweigs durch den UV-Reaktor kann der Anreicherungseffekt sogar zu einer integralen Meßgröße werden, wenn sich der Ionendetektor direkt im Wasser des UV-Reaktors befindet. Im Gegensatz zu der Anreicherung der nicht kohlenstoffhaltigen Zersetzungsprodukte im Wasser des UV-Reaktors wird vorzugsweise das bei der Zersetzung der organischen Verbindung entstandene Kohlendioxid kontinuierlich aus dem Wasser des UV-Reaktors ausgetrieben; es kann in der den UV-Reaktor verlassenden Gasphase mit einem Kohlendioxid-Detektor als differentieller Wert nachgewiesen werden.

Als Ionenaustauscher im Hauptstrom, der das für den Nachweis benötigte Wasser mit extrem hoher Reinheit stets wieder verfügbar macht, dient erfindungsgemäß eine Mischung aus stark saurem Kationenaustauscher und stark basischem Anionenaustauscher, die sowohl Kationen als auch Anionen aus dem zu reinigenden Kreislaufwasser entfernt. Prinzipiell kann in allen Verfahrensausführungen das den UV-Reaktor verlassende Kreislaufwasser vor dem Ionennachweis mit einem Ionendetektor noch über einen weiteren selektiven Ionenaustauscher geleitet werden, wodurch bestimmte Ionenarten vor deren Nachweis aus dem Kreislaufwasser entfernt werden. In allen Verfahrensvarianten ist also verallgemeinert neben zumindest einem Ionenaustauscher im Hauptstrom der Einsatz weiterer (auch ionenselektiver) Ionenaustauscher und/oder auch Ionendetektoren an gleichen und/oder verschiedenen Stellen des Wasserkreislaufs aus unterschiedlichen Gründen möglich.

Als UV-Reaktor sind alle Vorrichtungen geeignet, die ein Bestrahlen des erfindungsgemäß durchgeleiteten Kreislaufwassers mit ultraviolettem Licht z. B. mit einer Wellenlänge von 185 nm ermöglichen, wodurch eine photochemische Zersetzung der nachzuweisenden zersetzungsfahigen heteroatomhaltigen organischen Verbindungen in kohlenstoffhaltige und nicht kohlenstoffhaltige Zersetzungsprodukte erfolgt. Aus den organisch gebundenen Heteroatomen wie Chlor oder Fluor entstehen dabei bekanntlich ionogene Zersetzungsprodukte, die zum Erzielen der hohen Nachweisempfindlichkeit herangezogen werden.

Die Detektion der Ionen im Kreislaufwasser, das den UV-Reaktor verläßt, erfolgt bevorzugt über eine herkömmliche Leitfähigkeitsmessung oder ionenselektive Elektroden. In Fällen extremer Anforderungen an die Nachweisempfindlichkeit empfiehlt sich darüber hinaus zur Minimierung der Temperaturabhängigkeit der Leitfähigkeitsmessung - und damit zur weiteren Steigerung der Nachweisempfindlichkeit - eine aktive Thermostatisierung der Leitfähickeitsmeßzelle, z. B. mit Hilfe eines thermostatisierten Wasserbades. Grundsätzlich kann der Ionendetektor auch wie erwähnt mit dem UV-Reaktor derart kombiniert werden, daß der Nachweis von Ionen direkt im UV-Reaktor erfolgt.

Die zu untersuchenden gasförmigen Phasen und/oder Gasströme können Prozeßströme oder repräsentative Teilmengen eines großen Gasvolumens darstellen (z. B. Raumluft, Abgase); ebenso ist es möglich z. B. Strippgase zu untersuchen, die durch eine flüssige Phase geführt wurden und dabei aus dieser Phase möglicherweise auch heteroatomhaltige organische Verbindungen ausgetrieben haben. Es kann in diesem Fall vorteilhaft sein, den pH-Wert der flüssigen Phase vor dem Durchleiten des Trägergasstromes durch Zugeben von Säure oder Lauge so einzustellen, daß ggf. in der flüssigen Phase vorhandene ausgasbare anorganische Verbindungen (z. B. CO₂, NH₃) in Verbindungen überführt werden, die nicht mehr in den Trägergasstrom übergehen können.

Als Trägergasstrom wird bevorzugt Luft eingesetzt, die frei von kohlenstoffhaltigen Substanzen ist. Vorteilhaft ist jedoch ein von molekularem Sauerstoff freies Trägergas, vorzugsweise Stickstoff, oder ein Edelgas wie z. B. Helium oder Argon.

In DE-39 09 227 sind in den Figuren 6 bis 9 exemplarisch Quellen dargestellt und beschrieben, aus denen auch gemäß dieser Erfindung zu untersuchende Gasströme herrühren können.

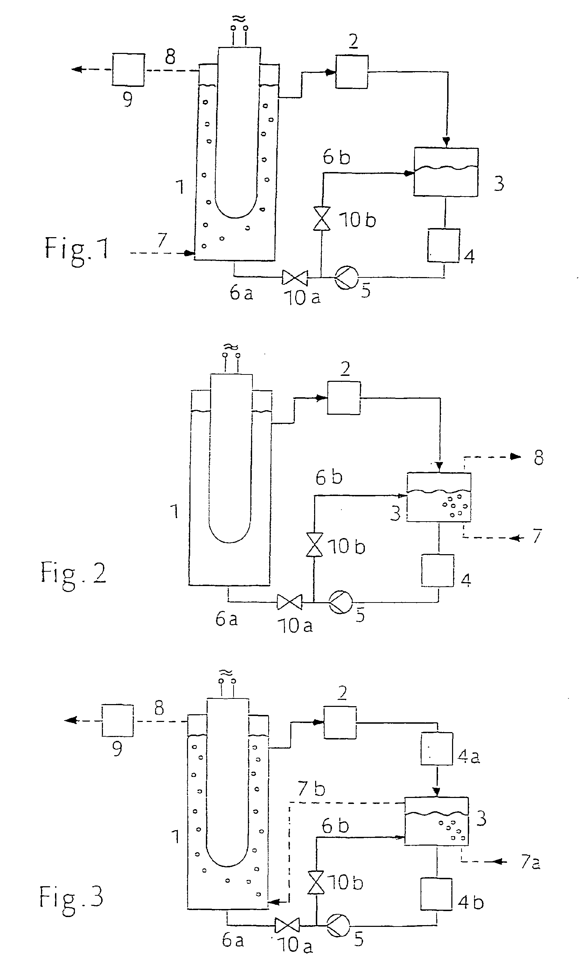

Das erfindungsgemäße Verfahren kann beispielsweise mittels der in den Figuren 1 bis 3 dargestellten Vorrichtungen durchgeführt werden.

In der Figur 1 besteht der Wasserkreislauf aus UV-Reaktor **1**, Ionendetektor **2**, Vorratsgefäß **3**, Ionenaustauscher **4**, Kreislaufpumpe **5** und den Regulierventilen **10a** und **10b.** Über die Kreislaufleitungen **6a** und **6b** wird das Kreislaufwasser ständig umgewälzt. Im unteren Teil des UV-Reaktors wird der zu untersuchende Gasstrom in das im UV-Reaktor befindliche Wasser über die Gas-Zuleitung **7** eingeperlt. Über die Gas-Ableitung **8** verläßt der Gasstrom den UV-Reaktor und kann ggf. durch einen Kohlendioxid-Detektor **9** geleitet werden. Die über den Gasstrom in den UV-Reaktor eingebrachten organischen Verbindungen werden in Kohlendioxid und ggf. nicht kohlenstoffhaltige Produkte zersetzt. Das entstehende Kohlendioxid wird mit dem Gasstrom, der durch das Wasser des UV-Reaktors geleitet wird, praktisch vollständig aus diesem Wasser entfernt und kann in dem den UV-Reaktor verlassenden Gasstrom nachgewiesen werden. Die nichtflüchtigen Zersetzungsprodukte werden im Kreislaufwasser, das den UV-Reaktor verläßt, mit dem Ionendetektor **2** nachgewiesen. Der Trennungseffekt kann durch Verlängern der Verweilzeit des Kreislaufwassers im UV-Reaktor verstärkt werden. In diesem Fall kann es vorteilhaft sein, den Ionendetektor direkt in das bestrahlte Wasser im UV-Reaktor einzusetzen. Mit Hilfe der Regulierventile **10a** und **10b** wird der Volumenstrom bzw. der Anteil des Kreislaufwassers, das den UV-Reaktor durchströmt bzw. an ihm vorbeigeführt wird, reguliert. Das den Ionendetektor verlassende Kreislaufwasser strömt zum Vorratsgefäß **3,** wird mit dem Wasser der Bypass-Leitung **6b** vereinigt und durchläuft anschließend den Ionenaustauscher **4,** durch den alle im Kreislaufwasser enthaltenen ionogenen Verbindungen aus diesem entfernt werden. Hinter dem Ionenaustauscher wird das ionenfreie Kreislaufwasser mit der Pumpe **5** zum Teil wieder über Leitung **6a** in den unteren Teil des UV-Reaktors sowie in die Bypass-Leitung **6b** gefördert.

In der Figur **2** ist der Wasserkreislauf aufgebaut wie in Figur 1, jedoch wird der zu untersuchende Gasstrom über die Gas-Zuleitung **7** in das im Vorratsgefäß **3** befindliche Kreislaufwasser eingeperlt. Über die Gas-Ableitung **8** verläßt der Gasstrom das Vorratsgefäß. Im Kreislaufwasser des Vorratsgefäßes lösen sich entsprechend der Lösungsgleichgewichte die Bestandteile des zugeführten Gasstromes. Die aus dem Gasstrom in Lösung gegangenen ionenbildenden Bestandteile (z. B. CO₂) werden über den Ionenaustauscher **4** aus dem Kreislaufwasser entfernt. Vor dem Ionenaustauscher können ggf. die Ionen mit einem Ionendetektor nachgewiesen werden. Die im Kreislaufwasser gelösten nichtionogenen Bestandteile werden teilweise mit dem Kreislaufwasser in den UV-Reaktor **1** gefördert. Die durch die UV-Bestrahlung aus den organischen Verbindungen entstandenen ionogenen Zersetzungsprodukte durchlaufen hinter dem UV-Reaktor den Ionendetektor **2** und werden hier nachgewiesen. Wird vor dem Ionendetektor, der beispielweise eine Leitfähigkeitsmeßzelle oder eine ionenselektive Elektrode sein kann, ein selektiver Ionenaustauscher durchlaufen, so können vor der Messung spezielle Ionenarten aus dem Kreislaufwasser entfernt werden. Über das Vorratsgefäß wird das Kreislaufwasser nach der Vereinigung der Teilströme wieder dem Ionenaustauscher zugeführt und hier von allen ionogenen Inhaltsstoffen befreit. Enthält der zu untersuchende Gasstrom keine ohne UV-Bestrahlung ionenbildenden Bestandteile, kann der Ionenaustauscher **4** auch direkt vor dem Vorratsgefäß **3** angeordnet sein, wobei allerdings die Vereinigung der Teilströme dann nicht im Vorratsgefäß, sondern vor dem Ionenaustauscher **4** erfolgen muß.

In Figur 3 wird der zu untersuchende Gasstrom nach dem Einleiten in das Wasser des Vorratsgefäßes über die Leitung **7a** mittels Leitung **7b** in das Wasser des UV-Reaktors eingeperlt. Über Leitung **8** verläßt der Gasstrom den UV-Reaktor und kann durch einen Kohlendioxid-Detektor **9** geleitet werden, um das bei der Zersetzung der organischen Verbindungen entstandene Kohlendioxid nachzuweisen. Die nach dem UV-Reaktor im Kreislaufwasser verbleibenden ionogenen Verbindungen werden mit dem Ionendetektor **2** nachgewiesen und können gegebenenfalls anschließend mit einem Ionenaustauscher **4a** bereits aus dem Kreislaufwasser entfernt werden. Enthält die in das Wasser des Vorratsgefäßes eingeleitete Gasphase u. a. in wäßriger Lösung ionenbildende Bestandteile, deren Löslichkeitsgleichgewicht praktisch vollständig auf der Seite der wäßrigen Lösung liegt (z. B. Chlorwasserstoff), so können diese im Wasser des Vorratsgefäßes aus der Gasphase entfernt werden. Diese nun im Kreislaufwasser gelösten ionogenen Bestandteile können separat detektiert werden, wenn der Ionenaustauscher **4a** alle ionogenen Zersetzungsprodukte zu beseitigen vermochte. Ansonsten werden alle ionogenen Inhaltsstoffe des Kreislaufwassers im Ionenaustauscher **4b** im Hauptstrom aus dem Kreislaufwasser entfernt, bevor dieses teilweise in den UV-Reaktor eingeleitet wird.

Das erfindungemäße Verfahren hat folgende Vorteile:
- Gasströme können kontinuierlich in einfacher Weise und mit hoher Empfindlichkeit auf heteroatomhaltige organische Inhaltsstoffe untersucht werden, selbst wenn diese nur in äußerst geringen Konzentrationen in der gasförmigen Phase vorliegen
- Durch das Verfahren können alle - kohlenstoffhaltige und nichtkohlenstoffhaltige - Zersetzungsprodukte der organischen Verbindungen nachgewiesen werden.
- Die Bestrahlungszeit des Kreislaufwassers im UV-Reaktor kann frei gewählt werden.
- Der totale organische Kohlenstoffgehalt (TOC) eines Gasstromes kann kontinuierlich bestimmt werden.
- Nichtflüchtige ionogene Zersetzungsprodukte (z. B. Chlorid-Ionen aus Chlorkohlenwasserstoffen) können im Wasser des UV-Reaktors in hohem Maße angereichert werden. Dadurch wird beispielsweise eine Nachweisgrenze für Chlorkohlenwasserstoffe, die in einer Gasphase vorliegen, erreicht, die im niedrigen ppt-Bereich liegt.
- Das für die Analyse als Lösemittel, Transportmittel und Reaktionspartner benötigte Wasser mit extrem hohem Reinheitsgrad ist durch den Kreistaufbetrieb ständig verfügbar.
- Die benutzten Vorrichtungen sind kalibrierfähig, wenn man eine Lösung verwendet. für die Konzentration und Art der enthaltenen zersetzungsfähigen Kohlenstoffverbindungen bekannt sind, und durch diese Lösung einen Trägergasstrom schickt, der anschließend in einer Vorrichtung z. B. entsprechend den Figuren 1 bis 3 untersucht wird.

Das erfindungsgemäße Verfahren wird durch folgende Beispiele erläutert, ohne darauf beschränkt zu sein:

### Beispiel 1

Verwendet wird die in Figur 1 beschriebene Vorrichtung. Bei eingeschaltetem UV-Strahler wird das Kreislaufwasser mit einem Volumenstrom von 0,5 l/h bei geöffnetem Regulierventil **10a** und geschlossenem Regulierventil **10b** umgepumpt. Die dem UV-Reaktor **1** zugeführte Gasphase (150 Norm-l/h) ist frei von zersetzungsfähigen organischen Verbindungen und enthält auch keine in Wasser ionenbildenden Stoffe Nach einer 24-stündigen Betriebszeit stellt sich die als Ionendetektor **2** verwendete Leitfähigkeitsmessung auf einen konstanten Meßwert von 0,08 µs/cm ein.

Der Volumenstrom des Kreislaufwassers wird bei geschlossenem Ventil **10b** stufenweise von 0,5 l/h auf 0,025 l/h reduziert und die sich einstellenden Leitfähigkeitswerte gemessen:

| **Volumenstrom Kreislaufwasser [l/h]** | **Leitfähigkeit [µs/cm]** |
|---|---|
| 0,500 | 0,08 |
| 0,200 | 0,20 |
| 0,100 | 0,31 |
| 0,025 | 0,52 |

Der oben beschriebene Versuch wird wiederholt, jedoch wird der Volumenstrom des Kreislaufwassers in Summe auf 0,5 l/h gehalten. Mit den Regulierventilen **10a** und **10b** werden die Teilmengen des Kreislaufwassers so eingestellt, daß der Volumenstrom über den UV-Reaktor **1** von 0,5 l/h stufenweise auf 0,025 l/h reduziert wird, wobei gleichzeitig der Volumenstrom, der am UV-Reaktor **1** und Ionendetektor **2** vorbei direkt in das Vorratsgefäß 3 eingeleitet wird, stufenweise von 0 l/h auf 0,475 l/h erhöht wird. Während der gesamten Versuchszeit wird der Ionenaustauscher **4** mit einem Volumenstrom von 0,5 l/h beaufschlagt.

Über den gesamten Einstellungsbereich wurde am Ionendetektor **2** eine konstante Leitfähigkeit von 0,08 µs/cm gemessen.

### Beispiel 2

Es wird wiederum die in Figur 1 beschriebene Vorrichtung verwendet. Die Regulierventile **10a** und **10b** werden so eingestellt, daß der Gesamtvolumenstrom von 1 l/h aufgeteilt wird, indem 0,025 l/h über die Leitung **6a** in den UV-Reaktor **1** gefahren werden und 0,975 l/h über Leitung **6b** in das Vorratsgefäß **3** zurücklaufen. Die dem UV-Reaktor **1** zugeführte Gasphase (150 Norm-l/h) ist frei von zersetzungsfähigen organischen Verbindungen und enthält auch keine in Wasser ionenbildenden Stoffe. Der sich einstellende Leitfähigkeitswert beträgt konstant 0,08 µs/cm. Die bisher zugeführte Gasphase wird gegen einen aus einem Phasenwechsler stammenden Gasstrom mit gleichem Mengenfluß getauscht. Der Phasenwechsler wird von einem Wasserstrom mit 10 l/h durchflossen, der Trichlormethan in einer Konzentration von 60 ppt, ansonsten aber keine in Wasser ionenbildenden Stoffe, enthält. Der Leitfähigkeitswert steigt von 0,08 µs/cm auf 0,13 µs/cm an. Damit wurde ein Chlorkohlenwasserstoff mit einer Konzentration im niedrigen ppt-Bereich nachgewiesen.

## Patentansprüche

1. Verfahren zum kontinuierlichen Nachweis von zersetzungsfähigen heteroatomhaltigen organischen Verbindungen, die in einer gasförmigen Phase vorliegen, bei dem das zu untersuchende Probegut in eine begrenzte Wassermenge eingebracht wird, die Bestandteil eines Wasserkreislaufs ist, der Kreislaufwasser über einen UV-Reaktor (**1**), in dem die zersetzungsfähigen heteroatomhaltigen organischen Verbindungen aus dein Probegut bestrahlt werden, über einen Ionendetektor (**2**), der zumindest die ionogenen Zersetzungsprodukte nachweist, und über einen Ionenaustauscher (**4**) führt,
**dadurch gekennzeichnet,**
**daß** aus dem Wasserkreislauf ein Teilstrom des Kreislaufwassers vor dem Eintritt in den UV-Reaktor (**1**) abgezweigt, am UV-Reaktor (**1**) und Ionendetektor (**2**) vorbeigeführt und vor dem oder am Eintritt in den Ionenaustauscher (**4**) wieder mit dem restlichen Kreislaufwasser vereinigt wird.

2. Verfahren nach Anspruch 1,
**dadurch gekennzeichnet,**
**daß** die Volumenströme der Kreislaufwasserströme durch den UV-Reaktor (**1**) und über den Ionenaustauscher (**4**) eingestellt werden können.

3. Verfahren nach Anspruch 1 oder 2,
**dadurch gekennzeichnet, daß** man
- das zu untersuchende gasförmige Probegut durch den mit Wasser gefüllten UV-Reaktor (**1**) leitet.

4. Verfahren nach Anspruch 1 oder 2,
**dadurch gekennzeichnet, daß** man
- das zu untersuchende gasförmige Probegut durch ein im Kreislauf befindliches Kreislaufwasservorratsgefäß (**3**) leitet, oder
- das zu untersuchende gasförmige Probegut durch das im Vorratsgefäß (**3**) enthaltene Wasser leitet und von dort durch das im UV- Reaktor (**1**) befindliche Wasser leitet.

5. Verfahren nach zumindest einem der vorherigen Ansprüche,
**dadurch gekennzeichnet, daß** man
- das Kreislaufwasser nach dem Zugeben des zu untersuchenden Probegutes und vor dem Durchleiten von Kreislaufwasser durch den UV-Reaktor (**1**) über einen Ionenaustauscher führt.

6. Verfahren nach zumindest einem der vorherigen Ansprüche,
**dadurch gekennzeichnet, daß** man
- das den UV-Reaktor (**1**) verlassende Kreislaufwasser zunächst durch einen selektiven Ionenaustauscher leitet und anschließend die Ionen im Kreislaufwasser dieses Teilstroms mit einem Ionendetektor nachweist.

7. Verfahren nach zumindest einem der vorherigen Ansprüche,
**dadurch gekennzeichnet, daß** man
- das zu untersuchende gasförmige Probegut herstellt, indem man einen Trägergasstrom, der frei von kohlenstoffhaltigen Verbindungen ist, durch eine flüssige Phase leitet, die die zersetzungsfähigen Kohlenstoffverbindungen enthält, wobei die flüchtigen heteroatomhaltigen organischen Verbindungen in den Trägergasstrom übergehen, und in dieser flüssigen Phase den pH-Wert auf einen Wert einstellt, bei dem der Trägergasstrom nur solche Verbindungen aus der flüssigen Phase ausstrippen kann, die ohne UV-Bestrahlung in wäßriger Lösung keine ionogenen Verbindungen bilden.

8. Verfahren nach zumindest einem der vorherigen Ansprüche,
**dadurch gekennzeichnet, daß** man
- ein von molekularem Sauerstoff freies Trägergas verwendet.

9. Verfahren nach zumindest einem der vorherigen Ansprüche,
**dadurch gekennzeichnet,**
**daß** die ionogenen Zersetzungsprodukte im Kreislaufwasser, das den UV-Reaktor (**1**) verläßt, mit Hilfe einer thermostatisierten Leitfähigkeitsmeßzelle nachgewiesen werden.

10. Vorrichtung zum kontinuierlichen Nachweis von zersetzungsfähigen heteroatomhaltigen organischen Verbindungen, die in einer gasförmigen organischen Phase vorliegen, wobei die Vorrichtung zumindest einen UV-Reaktor (**1**), einen Ionendetektor (**2**), einen Ionenaustauscher (**4**) und eine Umwälzpumpe (**5**) aufweist, die durch einen Wasserkreislauf miteinander in Verbindung stehen,
**gekennzeichnet durch**
- zumindest eine Kreislaufleitung (**6b**), die vor dem UV- Reaktor (**1**) aus dem Wasserkreislauf abzweigt, einen Teilstrom des Kreislaufwassers am UV-Reaktor (**1**) und Ionendetektor (**2**) vorbeileitet und den Teilstrom vor dem oder am Eintritt in den Ionenaustauscher (**4**) dem übrigen Kreislaufwasser wieder zuführt.

11. Vorrichtung nach Anspruch 10,
**gekennzeichnet durch**
- ein Regulierventil (**10a**) in der Kreislaufleitung (**6a**) die Kreislaufwasser zum UV-Reaktor (**1**) und Ionendetektor (**2**) leitet.

12. Vorrichtung nach Anspruch 10 oder 11,
**gekennzeichnet durch**
- eine thermostatisierte Leitfähigkeitsmeßzelle als Ionendetektor (**2**).

## Claims

1. A method for the continuous detection of decomposable heteroatom-containing organic compounds which are present in a gaseous phase, in which the sample material to be investigated is introduced into a limited amount of water which is part of a water circulation which passes circulated water via a UV reactor (**1**), in which the decomposable heteroatom-containing organic compounds from the sample material are irradiated, via an ion detector **(2),** which detects at least the ionic decomposition products, and via an ion exchanger (**4**), **characterized in that** a part-stream of the circulated water is removed from the water circulation before entering into the UV reactor **(1),** is caused to bypass the UV reactor **(1)** and ion detector **(2)** and is recombined with the remaining circulated water before or at the entrance into the ion exchanger **(4).**

2. A method according to claim 1, **characterized in that** the volume flow rate of the circulated water stream through the UV reactor **(1)** and via the ion exchanger **(4)** can be adjusted.

3. A method according to either of claims 1 and 2,
**characterized in that**
- the gaseous sample material to be investigated is passed through the UV reactor **(1)** filled with water.

4. A method according to either of claims 1 and 2,
**characterized in that**
- the gaseous sample material to be investigated is passed through a circulated water storage vessel **(3)** present in the circulation or
- the gaseous sample material to be investigated is passed through the water contained in the storage vessel **(3)** and from there is passed through the water present in the UV reactor **(1).**

5. A method according to at least one of the preceding claims, **characterized in that**
- the circulated water is fed via an ion exchanger after the addition of the sample material to be investigated and before the passage of the circulated water through the UV reactor **(1).**

6. A method according to at least one of the preceding claims, **characterized in that**
- the circulated water leaving the UV reactor (**1**) is first passed through a selective ion exchanger and the ions in the circulated water of this part-stream are then detected by means of an ion detector.

7. A method according to at least one of the preceding claims, **characterized in that**
- the gaseous sample material to be investigated is prepared by passing a carrier gas stream which is free of carbon-containing compounds through a liquid phase which contains the decomposable carbon compounds, the volatile heteroatom-containing organic compounds passing into the carrier gas stream, and adjusting the pH in this liquid phase to a value at which the carrier gas stream can strip out of the liquid phase only those compounds which form no ionic compounds in aqueous solution without UV irradiation.

8. A method according to at least one of the preceding claims, **characterized in that**
- a carrier gas free of molecular oxygen is used.

9. A method according to at least one of the preceding claims, **characterized in that** the ionic decomposition products in the circulated water which leaves the UV reactor **(1)** are detected with the aid of a thermostatted conductivity measuring cell.

10. An apparatus for the continuous detection of decomposable heteroatom-containing organic compounds which are present in a gaseous organic phase, the apparatus having at least a UV reactor (**1**), an ion detector (**2**), an ion exchanger (**4**) and a circulation pump **(5),** which are connected to one another by water circulation, **characterized by**
- at least one circulation line **(6b)** which branches from the water circulation upstream of the UV reactor **(1),** causes a part-stream of the circulated water to bypass the UV reactor **(1)** and ion detector **(2)** and feeds the part-stream back to the remaining circulated water before or at the entrance into the ion exchanger (**4**).

11. An apparatus according to claim 10, **characterized in that**
- a control valve (**10a**) in the circulation line **(6a)** passes the circulated water to the UV reactor **(1)** and ion detector **(2).**

12. An apparatus according to either of claims 10 and 11, **characterized by**
- a thermostatted conductivity measuring cell as ion detector (**2**).

## Revendications

1. Procédé d'identification en continu de composés organiques décomposables contenant des hétéroatomes et présentes dans une phase gazeuse, selon lequel on incorpore le produit à tester dans une quantité d'eau limitée faisant partie d'un circuit d'eau, lequel achemine de l'eau de circuit par un réacteur UV (1) dans lequel les composés organiques décomposables contenant des hétéroatomes sont irradiés hors du produit à tester, un détecteur d'ions (2) qui détecte au moins les produits de décomposition ionogènes, et un échangeur d'ions (4),
**caractérisé en ce qu'**
on dérive, du circuit d'eau, un flux partiel d'eau de circuit avant l'entrée dans le réacteur UV (1), on le fait passer au-delà du réacteur UV (1) et du détecteur d'ions (2) et on le réunit de nouveau avant l'échangeur d'ions (4) ou à l'entrée dans celui-ci avec l'eau de circuit restante.

2. Procédé selon la revendication 1,
**caractérisé en ce que**
les flux volumiques des flux d'eau de circuit peuvent être ajustés par le réacteur UV (1) et par l'intermédiaire de l'échangeur d'ions (4).

3. Procédé selon la revendication 1 ou 2;
**caractérisé en ce qu'**
on fait passer le produit gazeux à tester à travers le réacteur UV (1) rempli d'eau.

4. Procédé selon la revendication 1 ou 2,
**caractérisé en ce qu'**
- on fait passer le produit gazeux à tester à travers un réservoir d'eau de circuit (3) se trouvant dans le circuit, ou
- on fait passer le produit gazeux à tester à travers l'eau contenue dans le réservoir (3) et de là, on le fait passer à travers l'eau se trouvant dans le réacteur UV (1).

5. Procédé selon au moins l'une quelconque des revendications précédentes,
**caractérisé en ce qu'**
on achemine l'eau de circuit, après avoir ajouté le produit gazeux à tester et avant de la faire passer à travers le réacteur UV (1), par l'intermédiaire d'un échangeur d'ions.

6. Procédé selon au moins l'une quelconque des revendications précédentes,
**caractérisé en ce qu'**
on fait d'abord passer l'eau de circuit quittant le réacteur UV (1) à travers un échangeur d'ions sélectif, puis on détecte les ions dans l'eau de circuit de ce flux partiel avec un détecteur d'ions.

7. Procédé selon au moins l'une quelconque des revendications précédentes,
**caractérisé en ce qu'**
on prépare le produit gazeux à tester en faisant passer un flux de gaz porteur, qui est dépourvu de composés carbonés, à travers une phase liquide qui contient les composés carbonés décomposables, les composés organiques volatils contenant des hétéroatomes passant dans le flux de gaz porteur et, dans cette phase liquide, on ajuste le pH sur une valeur permettant au flux de gaz porteur d'éliminer par strippage de la phase liquide les composés qui ne forment pas de composés ionogènes sans irradiation UV dans une solution aqueuse.

8. Procédé selon au moins l'une quelconque des revendications précédentes
**caractérisé en ce qu'**
on utilise un gaz porteur dépourvu d'oxygène moléculaire.

9. Procédé selon au moins l'une quelconque des revendications précédentes,
**caractérisé en ce qu'**
on met les produits de décomposition ionogènes en évidence dans l'eau de circuit qui quitte le réacteur UV (1) à l'aide d'une cellule de mesure de conductivité thermostatée.

10. Dispositif de détection en continu de composés organiques décomposables contenant des hétéroatomes qui se présentent dans une phase gazeuse organique, le dispositif présentant au moins un réacteur UV (1), un détecteur d'ions (2), un échangeur d'ions (4) et une pompe de recirculation (5), qui sont reliés les uns aux autres par un circuit d'eau,
**caractérisé en ce qu'**
au moins une conduite de circuit (6b), qui part du circuit d'eau avant le réacteur UV (1), fait passer un flux partiel de l'eau de circuit au-delà du réacteur UV (1) et du détecteur d'ions (2) et recycle le flux partiel vers l'eau de circuit restante avant ou à l'entrée dans l'échangeur d'ions (4).

11. Dispositif selon la revendication 10,
**caractérisé en ce qu'**
une soupape de régulation (10a), dans la conduite de circuit (6a), conduit l'eau de circuit vers le réacteur UV (1) et le détecteur d'ions (2).

12. Dispositif selon la revendication 10 ou 11,
**caractérisé par**
une cellule de mesure de la conductivité thermostatée en tant que détecteur d'ions (2).
